# EUROPEAN PATENT APPLICATION

(11) **EP 0 619 313 A1**
(43) Date of publication of application: **12.10.1994**
(21) Application number: 94911773.3
(22) Date of filing: 01.10.1993
(51) Int. Cl.: C07D 307/62, A61K 31/375

(54) **LITHIUM SALT OF 2-O-ALKYLASCORBIC ACID**

(30) Priority: 02.10.1992 JP 264696/92
(71) Applicant: TSUMURA & CO., Chuo-ku, Tokyo 103 (JP)
(72) Inventor: SHIMIZU, Tadakazu, Toyonaka-shi, Osaka 560 (JP); KANEKO, Tatsuhiko, Mishima-gun, Osaka 618 (JP)
(74) Representative: Lewin, John Harvey
(86) International application number: JP9301409
(87) International publication number: WO9407883

(57) **Abstract**

A lithium salt of a 2-O-Alkylascorbic acid useful for preventing or treating circulatory function disorder.

## Description

### TECHNICAL FIELD

The present invention relates to a novel lithium salt of ascorbic acid derivatives.

### BACKGROUND ART

Recently, it has been revealed that active oxygen species or reactive organic radical species play an important role in aggravation of lesions (i.e., lowering of cell function, disturbances, destruction, necrosis, etc.) of diseases of heart, brain, kidney or the like, such as ischemic cardiac diseases, ischemic brain disorders, ischemic renal disorders, ischemic ulcer of the digestive system, cancer, arteriosclerosis, diabetes, cataract and the like (I. Fridovich, Annual Review of Pharmacology and Toxicology 23, 239 (1983); J. M. McCord, The New England Journal of Medicine 312, 159 (1985); K. P. Burton, J. M. McCord and G. Chai, American Journal of Physiology 246, H776 (1984); Masao Inoue et al., Active Oxygen and disease, Gakkai Shuppan Center (1992)). As the active oxygen species or reactive organic radical species in a living system are considered, among others, superoxide anion radical (O⁻·₂), hydroxyl radical (·OH), singlet oxygen (¹O₂), peroxide radical (ROO·) and the like. In particular, the relationship between the formation of O⁻·₂ in a living system and the subsequent damages of cells or tissues caused by the active oxygen species have important meanings. It is been known that superoxide dismutase effectively acts to scavenge O⁻·₂ effectively or specifically (D. N. Granger, G. Rutili, J. M. McCord, Gastroenterology 81, 22 (1981)). Also, it has been reported that such compounds as ascorbic acid, α-tocopherol, cysteine, reduced glutathione and the like have an activity to scavenge free radicals, and that these compounds could prevent lesions in tissues, which are seemed to be related with free radicals in pathological conditions (I. Fridovich, Science 201, 875 (1978)).

As ascorbic acid derivatives having prophylactic and therapeutic activity against functional disorders of the circulatory system, 2-O-ethers of ascorbic acid are described in JP-A 61-263969 (EP-A-202589).

### DISCLOSURE OF INVENTION

Based on the fundamental studies so far made, revealing that active oxygen species and organic radicals play a significantly important role in causing tissue disturbances in a living system, the present inventors have conducted research work for finding out a novel type of pharmaceutical which is more potent than the above free radical scavengers and excellent pharmacologically as well as pharmaceutically, aiming at scavenging active oxygen species and organic radicals. As the result, the present inventors found that novel lithium salts of 2-O-substituted ascorbic acid derivatives controlled, in experiments in vitro and in various animal test models, activity of lipid peroxidation enzyme, ischemic heart diseases, disturbances in cerebral function or the like in low doses. After further studies based on these findings, the present invention has been completed.

The present invention provides lithium salts of 2-O-alkylascorbic acids.

The lithium salt of 2-O-alkylascorbic acids is useful as a pharmaceutical composition for preventing and treating functional disorders of the circulatory system, or as an anticancer drug.

The present invention is illustrated below.

The lithium salt of 2-O-alkylascorbic acids of the present invention is a lithium salt of a compound of the formula (I): 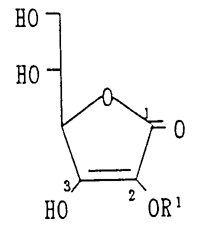
wherein R¹ represents alkyl. This salt may be any of mono-salt, di-salt and tri-salt.

The lithium may be attached to any oxygen atoms at the 1-, 2- or 3-position. Among them, the 3-position is preferred.

Examples of the alkyl group represented by R¹ in the above formula (I) include optionally substituted straight-chain or branched-chain alkyl groups.

The alkyl group in the optionally substituted straight-chain or branched-chain alkyl group is preferably that having 1 to 20 carbon atoms, more preferably that having 9 to 20 carbon atoms, particularly preferably straight-chain alkyl groups having 14 to 18 carbon atoms. Examples of the alkyl group include methyl, ethyl, n-propyl, isopropyl, n-butyl, isobutyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosyl and the like.

The number of methylene groups in the substituted straight-chain or branched-chain alkyl groups described above is preferably 1 to 19.

Examples of the substituent of the above alkyl group include an optionally substituted hydroxyl group, an optionally substituted amino group, an optionally substituted carboxyl group, an optionally substituted aminocarbonyl group, an optionally substituted vinyl group, an optionally substituted ethynyl group, optionally substituted cycloalkyl groups, optionally substituted aryl groups, optionally substituted heterocyclic groups, a quinoyl group of the formula: 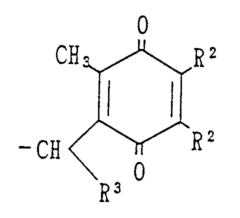
wherein R² is a methyl group or methoxy group, or the two R²'s may be joined together to form a group of the formula: -CH=CH-CH=CH-; and R³ is a phenyl, naphthyl, thienyl or pyridyl group each of which may optionally be substituted,
or a chroman-2-yl group of the formula: 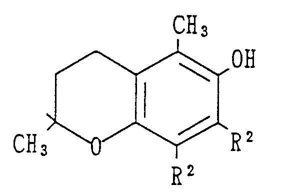
wherein each symbol is as defined above.

Examples of the optionally substituted hydroxyl group as the substituent of the above alkyl group include groups of the formula:

-O-R⁴

wherein R⁴ is hydrogen, C₁₋₃ alkyl or phenyl;
such as C₁₋₃ alkoxy (e.g., methoxy, ethoxy, propoxy, isopropoxy, etc.), phenoxy and the like in addition to an unsubstituted hydroxyl group.

Examples of the optionally substituted amino group as the substituent of the above alkyl group include groups of the formula: 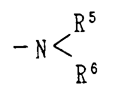
wherein R⁵ and R⁶ are the same or different and are hydrogen, C₁₋₃ alkyl, phenyl or p-hydroxyphenyl;
such as amino substituted with mono-C₁₋₃ or di-C₁₋₃ alkyl substituted amino (e.g., methylamino, dimethylamino, ethylamino, propylamino, isopropylamino, etc.), phenylamino, p-hydroxyphenylamino and the like in addition to an unsubstituted amino group.

Examples of the optionally substituted carboxyl as the substituent of the above alkyl include groups of the formula:

-CO-O-R⁷

wherein R⁷ is hydrogen, C₁₋₃ alkyl or phenyl;
such as methoxycarbonyl, ethoxycarbonyl, phenoxycarbonyl and the like in addition to an unsubstituted carboxyl group.

Examples of the optionally substituted aminocarbonyl group as the substituent of the above alkyl group include groups of the formula:

-CO-NH-R⁸

wherein R⁸ is hydrogen, C₁₋₃ alkyl, phenyl or p-hydroxyphenyl;
such as methylaminocarbonyl, ethylaminocarbonyl, isopropylaminocarbonyl, phenylaminocarbonyl, p-hydroxyphenylaminocarbonyl and the like in addition to an unsubstituted aminocarbonyl group.

Examples of the optionally substituted vinyl group as the substituent of the above alkyl group include groups of the formula: 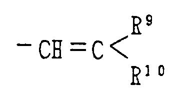
wherein R⁹ and R¹⁰ are the same or different and are hydrogen, C₁₋₅ alkyl, phenyl, p-methoxyphenyl, 3-pyridyl or 3,4-methylenedioxyphenyl;
such as propenyl, butenyl, pentenyl, hexenyl, heptenyl, 1,1-diphenylethenyl, 1-phenyl-1-(3-pyridyl)ethenyl, 1-phenyl-1-(2-thienyl)ethenyl and the like in addition to an unsubstituted vinyl group.

Examples of the optionally substituted ethynyl group as the substituent of the above alkyl group include groups of the formula:

-C≡C-R¹¹

wherein R¹¹ is hydrogen or C₁₋₆ alkyl;
such as methylethynyl, ethylethynyl, n-pentylethynyl and the like in addition to an unsubstituted ethynyl group.

Preferred examples of the cycloalkyl group of the optionally substituted cycloalkyl group as the substituent of the above alkyl group include cycloalkyl groups having 3 to 6 carbon atoms such as cyclopropyl, cyclobutyl, cyclopentyl, cyclohexyl and the like. The cycloalkyl group may have 1 to 3 substituents such as carboxyl, hydroxyl, C₁₋₃ alkyl or the like. Examples of the substituted cycloalkyl group include 1-carboxycyclopropyl, 2-carboxycyclopropyl, 1-carboxycyclopentyl, 1-carboxycyclohexyl, 4-carboxycyclohexyl and the like.

Examples of the optionally substituted aryl group as the substituent of the above alkyl group include groups of the formula: 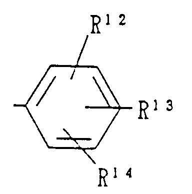
wherein R¹², R¹³ and R¹⁴ are the same or different and are hydrogen, C₁₋₃ alkyl, C₁₋₃ alkoxy, halogen, ethoxycarbonylethenyl, phenyl, carboxyl, carboxymethyl or 1-carboxyethyl;
and naphthyl optionally substituted with 1 to 3 substituents selected from C₁₋₃ alkyl, C₁₋₃ alkoxy, halogen, carboxy and acetyl. Concrete examples of the optionally substituted aryl include phenyl, 1- or 2-naphthyl, 2-, 3- or 4-monomethylphenyl, 2-, 3- or 4-monomethoxyphenyl, 2-, 3- or 4-monoethoxyphenyl, 2-, 3- or 4-monohalogenophenyl having a chlorine, bromine or fluorine atom as the halogen atom, 2,3-methylenedioxyphenyl, 3,4-dimethylphenyl, 3,4-dimethoxyphenyl, 4-(ethoxycarbonylethenyl)phenyl, 4-isopropylphenyl, 4-methoxycarbonylphenyl, 3,4,5-trimethylphenyl, 3,4,5-trimethoxyphenyl, 4-biphenylyl, 4-carboxyphenyl, 4-carboxymethylphenyl, 4-(1-carboxyethyl)phenyl and the like.

Examples of the heterocyclic group of the optionally substituted heterocyclic group as the substituent of the above alkyl group include 5 to 7 membered heterocyclic groups containing one sulfur atom, nitrogen atom or oxygen atom, 5 to 6 membered heterocyclic groups containing 2 to 4 nitrogen atoms, 5 to 6 membered heterocyclic groups containing 1 to 2 nitrogen atoms and one sulfur atom or oxygen atom and the like. Each of these heterocyclic groups may have 1 to 3 substituents such as C₁₋₃ alkyl optionally substituted with phenyl; carboxyl; hydroxyl; C₁₋₃ alkoxy; phenyl optionally substituted with halogen; halogen; carboxymethyl; benzoyl or the like. Concrete examples of the optionally substituted heterocyclic group include 2-, 3- or 4-pyridyl, 2- or 3-thienyl, morpholino, pyrrolidinyl, piperidino, piperidyl, piperazinyl, 4-phenylpiperazinyl, 4-(p-fluorophenyl)piperazinyl, 4-diphenylmethylpiperazinyl, 4-(p-methoxyphenyl)piperazinyl and the like.

Examples of the C₁₋₆ alkyl in the present specification include methyl, ethyl, n-propyl, isopropyl, n-butyl, isobutyl, n-pentyl, n-hexyl and the like.

Examples of the C₁₋₃ alkyl in the present specification include methyl, ethyl, n-propyl, isopropyl and the like.

Examples of the C₁₋₃ alkoxy in the present specification include methoxy, ethoxy, n-propoxy, isopropoxy and the like.

Examples of the halogen in the present specification include chlorine, bromine, fluorine and iodine.

The lithium salt of 2-O-alkylascorbic acids can be obtained by subjecting a compound of the above formula (I)(hereinafter referred to as compound (I)) to lithiation.

The above reaction can be carried out by conventional known methods, for example, by reaction with a lithium compound such as lithium carbonate or the like.

This reaction is carried out in a solvent which does not hinder the reaction. Examples of the solvent include water, alcohols (e.g., methanol, ethanol, etc.) and the like.

The amount of the lithium compound such as lithium carbonate or the like to be used is about 0.5 to 10 mol per mol of the compound (I) of the starting material.

The reaction temperature is about 10°C to 80°C.

The reaction time is about 10 minutes to 6 hours.

The 2-O-alkylascorbic acid lithium thus prepared can be isolated and collected by per se known separation and purification methods (e.g., chromatography using silica gel, polystyrene resin, active carbon, reversed phase or the like; recrystallization, etc.).

The compound (I) to be used as the starting material in the present invention can be prepared, for example, by the following reaction steps (JP-A 61-263969 (EP-A-202589), JP-A 60-130582 (US4780549), etc.). 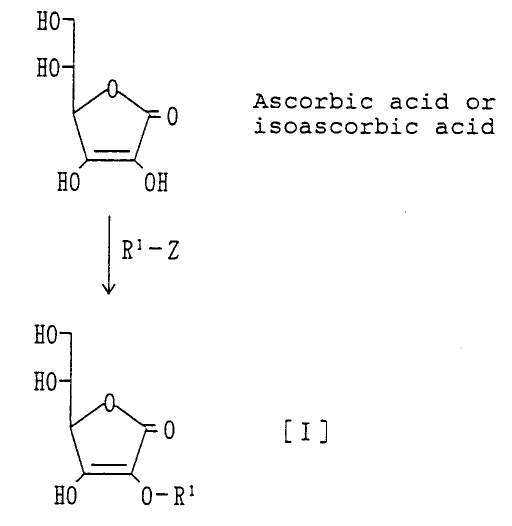
In the above process, the compound (I) can be prepared by reacting ascorbic acid or isoascorbic acid with a protecting agent such as methoxymethyl chloride, ethoxymethyl chloride, benzyl bromide, trimethylsilyl chloride, dimethyl-tert-butylsilyl chloride or the like according to per se known methods to obtain 3-O-ether, followed by reacton of the resulting 3-O-ether with a compound of the formula: R¹-Z wherein R¹ is as defined above and Z is halogen (e.g., chlorine, bromine, etc.). This reaction is carried out in the presence of an inorganic acid. Examples of the inorganic acid include alkaline metal hydroxides (e.g., sodium hydroxide, potassium hydroxide, etc.), alkaline metal salts (e.g., sodium carbonate, potassium carbonate, etc.) and the like. This reaction is carried out in a solvent which does not hinder the reaction. Examples of the solvent include amides (e.g., dimethylformamide, hexamethylphosphramide, etc.), sulfoxides (e.g., dimethyl sulfoxide, etc.), cyclic ethers (e.g., tetrahydrofuran, etc.) and the like. These solvents can be used alone or in combination of the two or more of them. The reaction temperature is about 1 to 18 hours. The reaction temperature is about 10 to 60°C.

The lithium salt of 2-O-alkylascorbic acids of the present invention shows free-radical scavenging and anticancer actions in experiments in vitro using lipid peroxidation enzyme, phospholipid hydrolase A₂ and GST-cdc25Hu2 (protein phosphatase), and also shows actions of preventing and improving cardiac functional disorders in an ischemia-reperfusion model in the heart of rats, while it shows remarkably low toxicity and no side effects. The compound of the present invention shows therapeutic, prophylactic and improving actions against various disorders, for example, ischemic cardiac disorders (arrhythmia, coronary arterial vasospasm, necrosis of cardiac tissue, myocardial infarction, etc.), subarachnoid hemorrhage disorders, ischemic disorders of cerebral tissue (cerebral infarction, dementia, senile dementia, etc.), ischemic renal disorders, ischemic disorders of digestive apparatuses (ulcer of digestive apparatuses, etc.), cancer, arteriosclerosis, diabetes, cataract or the like in mammals (e.g., mice, rats, rabbits, dogs, monkeys, humans, etc.), thus being useful as pharmaceutical compositions for preventing or treating functional disorders in the circulatory system or anticancer drug.

Specific examples of the use as the above preventing and improving composotion of the diseases include pharmaceutical compositions for improving the circulatory system such as pharmaceutical compositions of anti-arrhythmia, anti-myocardiac infarction, anti-cerebral infarction, preventing senile dementia, therapy and improvement after subarachnoid hemorrhage; pharmaceutical compositions for improving renal functions, pharmaceutical compositions for treating stress ulcer of digestive apparatuses; anticancer drugs and the like.

The compounds of the present invention are low in toxicity and can safely be administered orally or parenterally as pharmaceutical compositions (e.g., tablets, capsules including soft capsules and microcapsules, liquids, suppositories, injections, preparations for nasal inhalation, percutaneous preparations, eye drops, etc.) prepared by mixing the compound of the present invention with per se known pharmacologically acceptable carriers, excipients, diluents, etc. in accordance with per se known methods. While the dosage varies with the subjects, administration routes, symptoms, etc., it is usually, when orally administered to the above-mentioned mammals, about 0.1 mg/kg to 500 mg/kg body weight, preferably about 0.5 mg/kg to 200 mg/kg body weight 1 to 3 times a day.

When the compound is administered parenterally, for example, as a suppository, about 5 mg to 100 mg/kg in terms of the compound of the present invention, once or twice a day. As the injections, it is desirable to use, in terms of the compound of the present invention, about 0.1 mg/kg to 5 mg/kg once or twice a day.

For preparation of the above-mentioned compositions for oral use, a binder (e.g., hydroxypropylcellulose, hydroxymethylpropyl-methylcellulose, macrogol, etc.), a disintegrator (e.g., starch, carboxymethylcellulose calcium, etc.), an excipient (e.g., lactose, starch, etc.), a lubricant (e.g., magnesium stearate, talc, etc.) and the like may be suitably incorporated.

When a composition of parenteral use, for example, injectable preparation, an isotonizing agent (e.g., glucose, D-sorbitol, D-mannitol, sodium chloride, etc.), an antiseptic (e.g., benzyl alcohol, chlorobutanol, methyl parahydroxybenzoate, propyl parahydroxybenzoate, etc.), a buffer (e.g., phosphate buffer, sodium acetate buffer, etc.) and the like may be suitably incorporated.

### EXAMPLES

The following experiments and examples further illustrate the present invention in detail but are not to be construed to limit the scope thereof.

### Experiment 1

### Hemolysis and solubility of 2-O-octadecylascorbic acid lithium:

A physiological saline solution containing 2-O-octadecylascorbic acid lithium obtained in Example 1 below was warmed to 37°C, put in a Spitz roll, provided with rabbit defibrinated blood (Note)(0.1 ml) previously warmed to 37°C, shaken for mixing gently and warmed at 37°C for 1 hour. Then, this liquid was subjected to centrifugation. Macroscopic observation was made as to whether the supernatant had hemolysis.

As a result, hemolysis was not observed in an amount of not more than 1 µg/ml of 2-O-octadecylascorbic acid lithium.

### (Note) Rabbit defibrinated blood:

Blood (about 20 ml) was drawn from rabbits, put in a flask containing about twenty glass beads of diameter of 8 mm, shaken for mixing gently for about 5 minutes and filtered through gauze. This preparation was carried out before use.

The water-solubility was 1 g/83 ml (water) at 28°C.

### Experiment 2

### Toxicity of 2-O-octadecylascorbic acid lithium

### Method:

The test compound was administered orally or intravenously to Jcl:ICR mice and Jcl:Wistar rats (two mice or rats (male and female) per group). 2-O-octadecylascorbic acid lithium obtained in Example 1 as the test compound was suspended in 0.5% methyl cellulose in the case of oral administration and dissolved in physiological saline in the case of intravenous administration.

### Results:

In the both cases of the mice and rats, the LD₅₀ was not less than 2,000 mg/kg in the oral administration and in the region of 25 to 50 mg/kg in the intravenous administration. The differences between the sexes were not observed. Abnormal findings were not observed in the autopsy of the survivors. Pulmonary congestion was observed in the autopsy of the dead animals in the intravenous administration.

### Experiment 3

### Inhibitory activity against lipid peroxidation enzyme of 2-O-octadecylascorbic acid lithium

### Method:

Rat liver microsome (2 mg), which is a NADPH generating system, the test compound (2-O-octadecylascorbic acid lithium obtained Example 1)(300 µM) and carbon tetrachloride (20 mM) were reacted at 37°C for 12 minutes. A mixed solution of thiobarbituric acid and trichloroacetic acid was added to the solution. Absorbance was measured at 535 nm.

### Results:

The inhibitory ratio of the enzymatic activity was 75%. It was found that the test compound had free-radical scavenging activity.

### Experiment 4

### Inhibitory activity against phospholipase A₂ of 2-O-octadecylascorbic acid lithium

### Method:

Phospholipase A₂ derived from pig pancreas was reacted for 10 minutes in 0.1M glycine-NaOH buffer (pH 9) containing a test compound (2-O-octadecylascorbic acid lithium obtained in Example 1)(300 µM) and labeled phosphatidylcholine. Thereafter, Ca⁺⁺ (2.5 mM) was added and the reaction was continued for additional 5 minutes. The reaction was stopped by adding 0.2M EDTA solution to the reaction mixture. The mixture was extracted with acidic hexene. Scintillation of the hexene layer was measured.

### Results:

The enzymatic activity of the phospholipase was inhibited by 59%.

### Experiment 5

### GST-cdc25Hu2 (protein phosphatase) inhibitory activity of 2-O-octadecylascorbic acid lithium

A test compound (2-O-octadecylascorbic acid lithium obtained in Example 1)(80 µM) was added to a reaction mixture containing GST-cdc25Hu2 fused protein (60 µg/ml), p-nitrophenylphosphate (manufactured by Wako Pure Chemical Industries, Ltd.)(10 mM), bovine serum albumin (manufactured by Sigma, U.S.A.)(100 µg/ml), DDT (dithiothreitol) (manufactured by Wako Pure Chemical Industries, Ltd.)(10 mM) and HEPES (2-hydroxyethylpiperazine-N'-2-ethane sulfate)(25 mM, pH 8.0). The mixture was subjected to reaction at 37°C for 60 minutes. Then, absorbance at 405 nm was measured, and the inhibitory activity was evaluated based on the decrease of the absorbance.

### Results:

The cdc25 activity was inhibited by 93.6%. That is, anticancer activity was observed in the test compound.

### Experiment 6

### Inhibitory activity against development of myocardial infarction focus in rats of 2-O-octadecylascorbic acid

### Method:

Male Wistar-rats (11 to 12 weeks old) were used and anethesized with pentobarbital-Na, and medianus thoracotomy was carried out under artificial respiration. After incising the pericardium membrane, the heart was exposed. The base of the left coronary artery together with cardiac muscle was threaded with a sutural needle and thread (marketed by Lederle (Japan), Ltd., 7-0 silk), which were passed through a polyethylene tube, and the thread was pulled. Thus, coronary artery was obstructed. One hour later, the thread was loosed to make blood to flow again. Thirty to sixty minutes after reperfusion, the thorax was closed and the rat was kept under arousal.

After 24 hours, the rats were anesthetized again, and the heart was removed quickly and soaked in ice-cooled physiological saline. The coronary artery was obstructed again to know a dangerous region. Evans' Blue (1%) was perfused through a polyethylene tube inserted into the aorta retrogradely, and normal region was separated from the dangerous region. Thereafter, the left ventricle was divided into 6 parts parallel to the vertical axis and dyed with triphenyl tetrazolium chloride (TTC)(1%) at 37°C for 10 minutes except necrotic cells. The infarction focus was weighed. The test drug (2-O-octadecylascorbic acid obtained in Example 1) was dissolved in physiological saline, and administered intravenously 30 minutes after the coronary obstruction.

### Results:

In the group (N=12) of the intravenous administration of physiological saline (1 ml/kg), the infarction focus per the dangerous region (IS/AR) was 60.0±1.8%. On the other hand, the group (N=7) of the intravenous administration of the test compound (1 mg/kg) showed 40.5±4.6% (IS/AR) and significant inhibition of 32.7% was observed in comparison with the control group (P<0.001). There was no significant difference between the two groups in the dangerous region per left ventricle (AR/LV).

From the above results, it has become clear that the test compound significantly inhibits the development of myocardial infarction focus after coronary obstruction-reperfusion in rats in a dose of 1 mg/kg i.v.

### Example 1

2-O-octadecylascorbic acid (4.28 g, 10 mM) was suspended in water (150 ml). Lithium carbonate (0.37 g, 5mM) was added little by little with stirring. Then, ethanol (50 ml) was added to obtain a transparent homogeneous solution. The solution was subjected to spontaneous filtration. The ethanol was distilled off under reduced pressure, and the aqueous solution was lyophilized to obtain powder. It was washed with acetone, dried under reduced pressure in a desiccator to obtain 2-O-octadecylascorbic acid lithium (4.0 g)(mp 163-165°C).

### Example 2

Tablets containing 2-O-octadecylascorbic acid lithium (3 mg) and having the following formulation were prepared by a per se known method.

| | |
|---|---|
| 2-O-Octadecylascorbic acid lithium | 3 mg |
| Lactose | 65 mg |
| Corn starch | 40 mg |
| Hydroxypropylcellulose | 2 mg |
| Magnesium stearate | 0.2 mg |
| | 110.2 mg (per tablet) |

### Example 3

Tablets containing 2-O-octadecylascorbic acid lithium (500 mg) and having the following formulation were prepared by a per se known method.

| | |
|---|---|
| 2-O-Octadecylascorbic acid lithium | 500 mg |
| Starch isomerized to α-isomer | 15 mg |
| Corn starch | 22 mg |
| Magnesium stearate | 3 mg |
| | 540 mg (per tablet) |

## Claims

1. A lithium salt of 2-O-alkylascorbic acid.

2. A lithium salt of 2-O-alkylascorbic acid according to claim 1, wherein the alkyl has 14 to 18 carbon atoms.

3. A pharmaceutical composition for preventing or treating a functional disorder of the circulatory system comprising a lithium salt of 2-O-alkylascorbic acid.
